# EUROPEAN PATENT APPLICATION

(11) **EP 3 508 291 A1**
(43) Date of publication of application: **10.07.2019**
(21) Application number: 17842504.7
(22) Date of filing: 25.07.2017
(51) Int. Cl.: B22F 9/24, C09K 11/08, A61K 47/02, A61K 41/00, G01N 21/64

(54) **METHOD FOR PREPARING SOLUTION CONTAINING LIGAND-BONDED GOLD NANOCLUSTERS**

(30) Priority: 23.08.2016 US 201662378303 P
(71) Applicant: Goldred Nanobiotech Co., Ltd., Taoyuan, Taiwan 32057 (TW)
(72) Inventor: CHANG, Hongshong, Taoyuan Taiwan 32057 (TW); LIN, Chengan, Taoyuan Taiwan 32023 (TW); LIN, Liangchih, Taoyuan Taiwan 32057 (TW); HUANG, Zihyun, Taoyuan Taiwan 32057 (TW); LI, Kuanjung, Taoyuan Taiwan 32057 (TW); HOU, Tzuyin, Taoyuan Taiwan 32023 (TW); CHUNG, Yuhsuan, Taoyuan Taiwan 32057 (TW)
(74) Representative: Zeitler Volpert Kandlbinder Patent- und Rechtsanwälte Partnerschaft mbB
(86) International application number: PCT/CN2017/000411
(87) International publication number: WO 2018/036078

(57) **Abstract**

The present invention discloses a process for forming a solution containing gold nanoclusters binding with ligands, the process comprises the following steps: provide a aqueous solution that comprises a gold precursor, a base and ligands ;perform a reduction reaction by adding a reductant into the aqueous solution to form a liquid containing gold nanoclusters binding with the ligands ;concentrate the liquid containing the gold nanoclusters binding with the ligands to a solid; dissolve the solid into water to form a crude solution; and perform a purification process by passing the crude solution through a membrane or a dialysis tube to obtain the solution containing the gold nanoclusters binding with the ligands.

## Description

### BACKGROUND OF THE INVENTION

### 1. Field of the Invention

The present invention generally relates to a process for forming a solution containing gold nanoclusters binding with a ligand. In particular, the ligand comprises lipoic acid and dihydrolipoic acid.

### 2. Description of the Prior Art

In modern biological analysis, various kinds of organic dyes are used. However, with each passing year, more flexibility is being required of these dyes, and the traditional dyes are often unable to meet the expectations. To this end, semiconductor quantum dots have quickly filled in the role, being found to be superior to traditional organic dyes on several counts, one of the most immediately obvious being brightness (owing to the high quantum yield) as well as their stability (much less photo-bleaching).

The use of semiconductor quantum dots for highly sensitive cellular imaging has seen major advances over the past decade. The improved photostability of semiconductor quantum dots for example, allows the acquisition of many consecutive focal-plane images that can be reconstructed into a high-resolution three-dimensional image. Another application that takes advantage of the extraordinary photostability of quantum dot probes is the real-time tracking of molecules and cells over extended periods of time.

Semiconductor quantum dots have also been employed for in vitro imaging of pre-labeled cells. The ability to image single-cell migration in real time is expected to be important to several research areas such as embryogenesis, cancer metastasis, stem-cell therapeutics, and lymphocyte immunology.

But there is a remaining issue with semiconductor quantum dot probes containing toxic ions, such as Cadmium and Lead. For this reason, we have been used fluorescent gold nanoclusters, so-called gold- quantum dots, instead of semiconductor quantum dots, wherein gold- quantum dots is nontoxic, having biocompatibility and high fluorescence quantum yield. Moreover, it is confirmed that gold-quantum dots is able to process different fluorescence colors by changing size thereof.

However, it is really difficult to synthesize gold- quantum dots. Gold- quantum dots are from PAMAM- encapsulated Au generally, wherein the PAMAM dendrimer is costly and gold-quantum dots are unable to be mass production at once.

Therefore, in view of the above mentioned problems, a novel process for preparing gold- quantum dots and also the related derivatives is an important research topic in industry.

### SUMMARY OF THE INVENTION

According to the above, the present invention provides a novel process for forming a solution containing gold nanoclusters binding with ligands to fulfill the requirements of this industry.

One object of the present invention is to discloses a novel process for forming a solution containing gold nanoclusters binding with ligands, the process comprises the following steps: provide a aqueous solution that comprises a gold precursor, a base and ligands; perform a reduction reaction by adding a reductant into the aqueous solution to form a liquid containing gold nanoclusters binding with the ligands; concentrate the liquid containing the gold nanoclusters binding with the ligands to a solid at 30-60°C; dissolve the solid into water to form a crude solution; and perform a purification process by passing the crude solution through a membrane or a dialysis tube to obtain the solution containing the gold nanoclusters binding with the ligands.

The invention process is a one-batch process. A key feature of the invention process is to form the gold nanoclusters binding with the ligands in the aqueous phase in only one step. Secondly, the invention process only uses water as the medium, so the process is an environmental-friendly process. Moreover, the gold nanoclusters binding with the ligands prepared by the invention process do not contain any harmful or toxic solvents such as toluene or dimethylformamide, as a result, the gold nanoclusters binding with the ligands prepared by the invention process are very suitable for cosmetic and medical applications.

### BRIEF DESCRIPTION OF THE DRAWINGS

FIG. 1 is the TEM photo of the solution containing gold nanoclusters binding with lipoic acid ligands of the example 1 in the present invention;
FIG. 2 is the TEM photo of the single gold nanocluster binding with lipoic acid ligands of the example 1 in the present invention;
FIG. 3 is the core size distribution of the gold nanoclusters binding with lipoic acid ligands of the example 1 in the present invention; FIG. 3 is calculated from FIG. 2 by software;
FIG. 4 is the size distribution by number of the gold nanoclusters binding with lipoic acid ligands; FIG. 4 is measured by DLS;
FIG. 5 is the size distribution by volume of the gold nanoclusters binding with lipoic acid ligands; FIG. 5 is measured by DLS;
FIG. 6 is the X-ray photoelectron spectrum of the gold nanoclusters binding with lipoic acid ligands of the example 1 in the present invention ;
FIG. 7 is the TGA diagram of the gold nanoclusters binding with lipoic acid ligands of the example 1 in the present invention;
FIG. 8 is the FTIR spectrum of the gold nanoclusters binding with lipoic acid ligands of the example 1 in the present invention;
FIG. 9 is XRD pattern of the gold nanoclusters binding with lipoic acid ligands of the example 1 in the present invention;
FIG. 10 illustrates the relation between fluorescent strength of the gold nanoclusters binding with lipoic acid ligands and heating temperature
FIG. 11 illustrates the relation between fluorescent strength of the gold nanoclusters binding with lipoic acid ligands and UV treatment ;and
FIG. 12 illustrates the relation between fluorescent strength of the gold nanoclusters binding with lipoic acid ligands and the concentration of the solution containing the gold nanoclusters binding with lipoic acid ligands

### DESCRIPTION OF THE PREFERRED EMBODIMENTS

What is probed into the invention is a fluorescent gold nanocluster and method for forming the same. Detail descriptions of the structure and elements will be provided in the following in order to make the invention thoroughly understood. Obviously, the application of the invention is not confined to specific details familiar to those who are skilled in the art. On the other hand, the common structures and elements that are known to everyone are not described in details to avoid unnecessary limits of the invention. Some preferred embodiments of the present invention will now be described in greater detail in the following specification. However, it should be recognized that the present invention can be practiced in a wide range of other embodiments besides those explicitly described, that is, this invention can also be applied extensively to other embodiments, and the scope of the present invention is expressly not limited except as specified in the accompanying claims.

Having summarized various aspects of the present invention, reference will now be made in detail to the description of the invention as illustrated in the drawings. While the invention will be described in connection with these drawings, there is no intent to limit it to the embodiment or embodiments disclosed therein. On the contrary the intent is to cover all alternatives, modifications and equivalents included within the spirit and scope of the invention as defined by the appended claims.

It is noted that the drawings presents herein have been provided to illustrate certain features and aspects of embodiments of the invention. It will be appreciated from the description provided herein that a variety of alternative embodiments and implementations may be realized, consistent with the scope and spirit of the present invention.

It is also noted that the drawings presents herein are not consistent with the same scale. Some scales of some components are not proportional to the scales of other components in order to provide comprehensive descriptions and emphasizes to this present invention.

A representative embodiment of the present invention discloses a process for forming a solution containing gold nanoclusters binding with ligands, the process comprises the following steps: provide a aqueous solution that comprises a gold precursor, a base and ligands ;perform a reduction reaction by adding a reductant into the aqueous solution to form a liquid containing gold nanoclusters binding with the ligands ;concentrate the liquid containing the gold nanoclusters binding with the ligands to a solid at 30-60°C; dissolve the solid into water to form a crude solution; and perform a purification process by passing the crude solution through a membrane or a dialysis tube to obtain the solution containing the gold nanoclusters binding with the ligands.

In one preferred example of the representative embodiment, the process further comprises performing a heating process and/or a UV treatment to increase the fluorescent strength of the solution containing the gold nanoclusters binding with the ligands.

In one example of the representative embodiment, the heating process is performed at a temperature between 30 and 150°C.

In one example of the representative embodiment, the UV treatment is performed at a wavelength of 300-400nm.

In one example of the representative embodiment, the gold precursor comprises Au(III) ions. Preferably, the gold precursor is AuCl₃ or HAuCl₄.

In one example of the representative embodiment, the mole ratio of the gold precursor to the ligands is less than 10, and the ligands comprise lipoic acid and dihydrolipoic acid.

In one example of the representative embodiment, the base comprises NaOH and KOH.

In one example of the representative embodiment, the reductant comprises : Sodium borohydride, Sodium citrate, Potassium bitartrate, Dithiothreitol, Tris(2-carboxyethyl)phosphine, Tetrabutylammonium nitrate, ascorbic acid, glutathione. Preferably, the reductant is Sodium borohydride.

In one example of the representative embodiment, the reduction reaction is performed at 5-40°C.

In one example of the representative embodiment, the purification process is applied for keeping nanoclusters having a molecular weight between 10 and 100kDa.

In one example of the representative embodiment, the gold nanoclusters binding with ligands are characterized with a Fourier transform infrared spectrum comprising bands at 3261, 2920, 2852, 1560 and 1401 cm⁻¹.

In one example of the representative embodiment, the gold nanoclusters binding with ligands are character ized with an X-ray powder diffraction pattern comprising peaks at 38.5° (111), 44.6° (200), 64.8° (220), and 77.8° (311) 2-theta degree.

In one example of the representative embodiment, the gold nanoclusters binding with ligands have a hydrodynamic diameter average size between 1 and 4nm.

In one example of the representative embodiment, the gold nanoclusters binding with the ligands have a weight ratio of gold to the ligands between 0.5 and 10.

In one example of the representative embodiment, the gold nanoclusters binding with the ligands, being a part of one comprises cosmetic composition, food composition and pharmaceutical composition.

Accordingly, the invention process has the following advantages. The invention process is a one-batch process and easy to scale up. A key feature of the invention process is to form the gold nanoclusters binding with the ligands in the aqueous phase in only one step. Secondly, the invention process only uses water as the medium, so the process is a green process. Moreover, the gold nanoclusters binding with the ligands prepared by the invention process do not contain any harmful or toxic solvents such as toluene or dimethylformamide, as a result, the gold nanoclusters binding with the ligands prepared by the invention process are very suitable for cosmetic and medical related applications.

### Example 1 : The invention process for preparing a solution containing gold nanoclusters binding with lipoic acid ligands.

30µmol of lipoic acid was dissolved in DI water containing sodium hydroxide. 10µmol of gold (III) chloride trihydrate was added under stirring at room temperature. Sodium borohydride was added as reducing agent, then the mixture was stirred for 15hrs at room temperature. The reaction mixture was concentrated to solid under 55°C , then dissolve by DI water to form crude solution. Free ligands in crude solution was purified by applying 10kDa membrane filtration device. A solution containing gold nanoclusters binding with lipoic acid ligands was prepared.

The gold nanoclusters binding with lipoic acid ligands prepared by the procedure described in example 1 are characterized by Transmission electron microscopy (TEM), dynamic light scattering (DLS), X-ray photoelectron spectroscopy(XPS), thermogravimetric analysis (TGA), Fourier transform infrared spectrometer (FTIR) and X-ray diffraction (XRD).

The typical parameters of the gold nanoclusters binding with lipoic acid ligands prepared by the procedure described in example 1 are listed in TABLE 1.

**TABLE 1**

| Parameter | Method | Results |
|---|---|---|
| Size of gold core | TEM | 1.45±0.34nm |
| Average Size by number | DLS | 2.27±0.45nm |
| Shape | TEM | Sphere |
| Surface chemistry | XPS | Atom(%): C(73.9%); 0(17.0%) |
| | | S(4.4%);Na(2.7%);N(1.3%);Au(0.6%) |
| Surface charge | Zeta-potential | -55.4±2.9mV |
| Chemical composition | TGA/ FTIR | Gold core: 67.39% |
| | (Dry sample) | Lipoic acid: 32.61% |
| Gold concentration in the solution | ICP-MS | 1560ppm |
| Purity | ICP-MS | 99.81% |
| Crystal structure | XRD | Cubic |
| Partition coefficient | ICP-MS | logP (_{octanol/water}) : -1.10 |

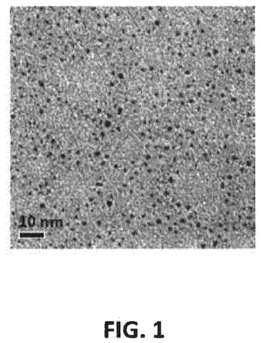

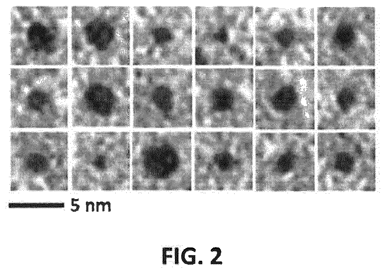

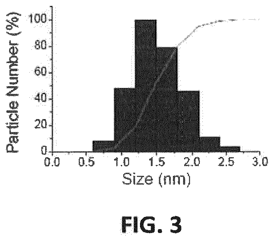

As shown in FIG. 1 and FIG. 2, TEM analysis show that the gold nanoclusters binding with lipoic acid ligands has a size less than 10nm and well dispersed in the aqueous solution. FIG. 3 indicated that the gold nanoclusters binding with lipoic acid ligands have an average core diameter being 1.45+0.34nm.

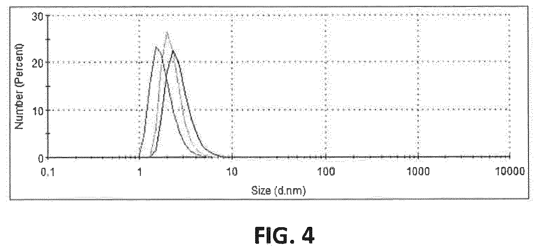

As shown in FIG. 4, DLS analysis showed the size distribution by number for 3 lots of the gold nanoclusters binding with lipoic acid ligands. The data is 1.82nm with standard deviation of 0.56nm, 2.28nm with standard deviation of 0.60nm, and 2.71nm with standard deviation of 0.89nm, respectively.

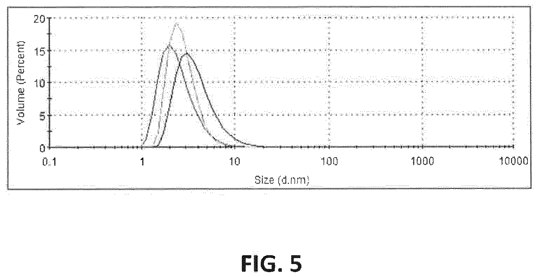

As shown in FIG 5. DLS analysis showed the size distribution by volume for 3 lots of the gold nanoclusters binding with lipoic acid ligands. The data is 2.56nm with standard deviation of 1.44nm, 2.80nm with standard deviation of 0.96nm, and 4.00nm with standard deviation of 2.16nm, respectively.

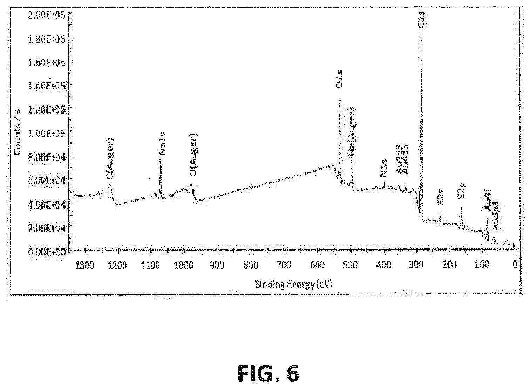

As shown in FIG. 6, X-ray photoelectron spectroscopy showed the atom percent of C, O, S, Na, N and Au is 73.9%, 17.0%, 4.4%, 2.7%, 1.3%, 0.6% respectively.

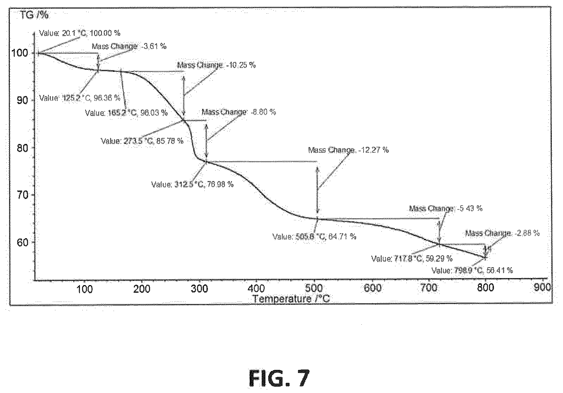

As shown in FIG. 7, Thermogravimetric analysis showed the weight percent of the gold and the ligands is 67.39% and 32.61%.

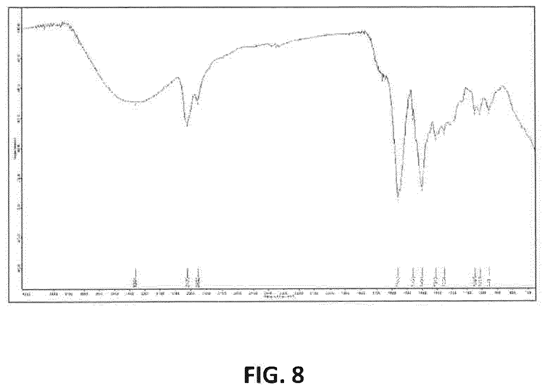

As shown in FIG. 8, Fourier transform infrared spectrum indicated bands at 3261, 2920, 2852, 1560 and 1401 cm⁻¹.

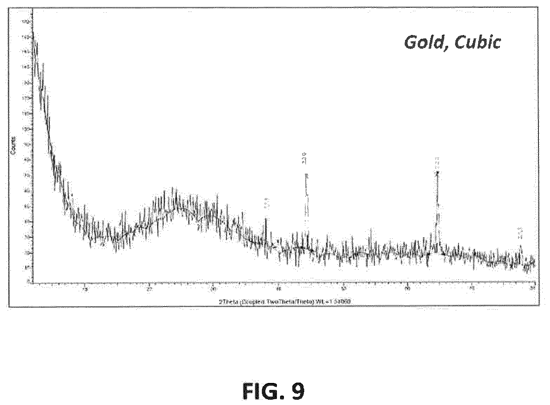

As shown in FIG. 9, X-ray diffraction showed diffraction pattern with four distinct diffraction peaks at 38.5°(111), 44.6°(200), 64.8°(220), and 77.8°(311)

**The process parameter related to the fluorescent strength of the gold nanoclusters binding with lipoic acid ligands.**

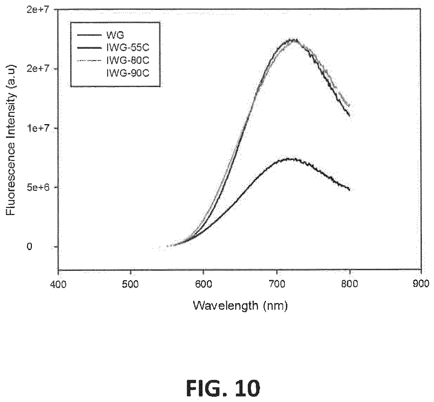

As shown in FIG. 10, WG represents the invention process without performing concentrating procedure; IWG-55C, IWG-80C and IWG-90C represents the invention process with performing the concentrating procedure and a further heating procedure at 55°C, 80 °C and 90°C respectively. Obviously, the heating procedure is able to increase the fluorescent strength of the gold nanoclusters binding with lipoic acid ligands at wavelength of 700nm.

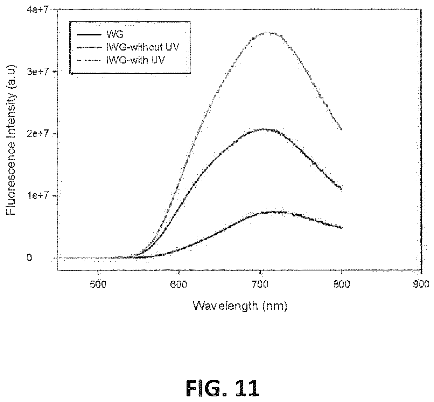

As shown in FIG. 11, WG represents the invention process without performing concentrating procedure; IWG-UV represents the invention process with performing the concentrating procedure and a further UV treatment at 365nm. Obviously, the UV treatment is able to increase the fluorescent strength of the gold nanoclusters binding with lipoic acid ligands at wavelength of 700nm.

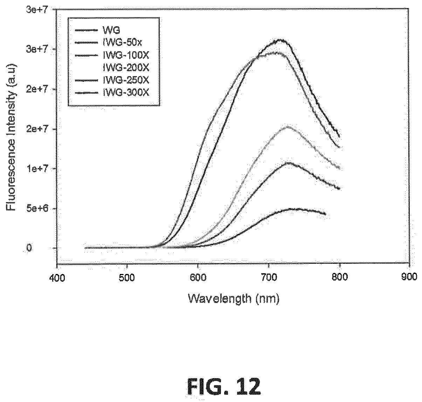

As shown in FIG. 12, WG represents the invention process without performing concentrating procedure; IWG-50X, IWG-100X, IWG-200X, IWG-250X and IWG-300X represent the invention process with performing concentrating procedure to increase the concentration of the gold nanoclusters binding with lipoic acid ligands to 50 folds, 100folds, 200folds, 250folds and 300folds of the original concentration respectively. When the concentration of the gold nanoclusters binding with lipoic acid ligands increases, the fluorescent intensity increases. For the purpose to maximize the fluorescent strength of the gold nanoclusters binding with lipoic acid ligands, the invention process have to concentrate the liquid containing the gold nanoclusters binding with the ligands to a solid and dissolve the solid again for further purification. Accordingly, the solid state after the claimed concentrating step is a key in the present invention.

Obviously many modifications and variations are possible in light of the above teachings. It is therefore to be understood that within the scope of the appended claims the present invention can be practiced otherwise than as specifically described herein. Although specific embodiments have been illustrated and described herein, it is obvious to those skilled in the art that many modifications of the present invention may be made without departing from what is intended to be limited solely by the appended claims.

## Claims

1. A process for forming a solution containing gold nanoclusters binding with ligands, the process comprising:
providing a aqueous solution that comprises a gold precursor, a base and ligands;
performing a reduction reaction by adding a reductant into the aqueous solution to form a liquid containing gold nanoclusters binding with the ligands;
concentrating the liquid containing the gold nanoclusters binding with the ligands to a solid at 30-60°C;
dissolving the solid with water to form a crude solution; and
performing a purification process by passing the crude solution through a membrane or a dialysis tube to obtain the solution containing the gold nanoclusters binding with the ligands.

2. The process of claim 1 further comprises a heating process and/or a UV treatment to increase the fluorescent strength of the solution containing the gold nanoclusters binding with the ligands.

3. The process of claim 2, wherein the heating process is performed at a temperature between 30 and 150°C.

4. The process of claim 2, wherein the UV treatment is performed at a wavelength of 300-400nm.

5. The process of claim 1, wherein the gold precursor comprises Au(III) ions.

6. The process of claim 1, wherein the mole ratio of the gold precursor to the ligands is less than 10, and the ligands comprise lipoic acid and dihydrolipoic acid.

7. The process of claim 1, wherein the base comprises NaOH and KOH.

8. The process of claim 1, wherein the reductant comprises : Sodium borohydride, Sodium citrate, Potassium bitartrate, Dithiothreitol, Tris(2-carboxyethyl)phosphine, Tetrabutylammonium nitrate, ascorbic acid, glutathione.

9. The process of claim 1, wherein the reduction reaction is performed at 5-40°C.

10. The process of claim 1, wherein the purification process is applied for keeping nanoclusters having a molecular weight between 10 and 100kDa.

11. The process of claim 1, wherein the gold nanoclusters binding with ligands are characterized with a Fourier transform infrared spectrum comprising bands at 3261, 2920, 2852, 1560 and 1401 cm⁻¹.

12. The process of claim 1, wherein the gold nanoclusters binding with ligands are characterized with an X-ray powder diffraction pattern comprising peaks at 38.5° (111), 44.6° (200), 64.8° (220), and 77.8° (311) 2-theta degree.

13. The process of claim 1, wherein the gold nanoclusters binding with ligands have a hydrodynamic diameter average size between 1 and 4nm.

14. The process of claim 1, wherein the gold nanoclusters binding with the ligands have a weight ratio of the gold to the ligands between 0.5 and 10.

15. The process of claim 1, wherein the gold nanoclusters binding with the ligands, being a part of one comprises cosmetic composition, food composition and pharmaceutical composition.
